# EUROPÄISCHE PATENTSCHRIFT

(11) **EP 2 934 279 B1**
(45) Veröffentlichungstag und Bekanntmachung des Hinweises auf die Patenterteilung: **30.01.2019**
(21) Anmeldenummer: 13828771.9
(22) Anmeldetag: 12.12.2013
(51) Int. Cl.: A61B 1/06, A61B 90/30, A61B 34/30, A61B 90/00, A61B 1/00, A61B 1/05, A61B 1/313, A61B 17/34, A61B 1/32

(54) **ENDOSKOP UND CHIRURGISCHES ROBOTERSYSTEM MIT EINEM MEHRKAMERASYSTEM FÜR DIE MINIMAL-INVASIVE CHIRURGIE**
ENDOSCOPE AND SURGICAL ROBOTER SYSTEM WITH A MULTI-CAMERA SYSTEM FOR MINIMALLY INVASIVE SURGERY
ENDOSCOPE ET SYSTÈME ROBOTIQUE CHIRURGICAL DOTÉS D'UN SYSTÈME MULTICAMÉRA POUR LA CHIRURGIE MINIMALEMENT INVASIVE

(30) Priorität: 20.12.2012 DE 102012025102
(43) Veröffentlichungstag der Anmeldung: 28.10.2015
(73) Patentinhaber: avateramedical GmbH, 07745 Jena (DE)
(72) Erfinder: VON GRÜNBERG, Hubertus, 30625 Hannover (DE); SEEBER, Marcel, 07745 Jena (DE)
(74) Vertreter: Ascherl, Andreas
(86) Internationale Anmeldenummer: PCT/DE2013/000805
(87) Internationale Veröffentlichungsnummer: WO 2014/094718

(56) Entgegenhaltungen:
- WO-A2-2007/146987
- WO-A2-2009/057117
- US-A1- 2005 234 294
- US-A1- 2011 306 832

## Beschreibung

Die vorliegende Erfindung betrifft ein Endoskop mit einem Mehrkamerasystem für den Einsatz bei minimal-invasiven Eingriffen sowie einem entsprechenden chirurgischen Roboter, vor allem für den Einsatz in der minimal-invasiven Chirurgie, wie der Laparoskopie.

Minimal-invasive chirurgische Eingriffe, wie laparoskopische Operationen, erfolgen über den Einsatz von chirurgischen Instrumenten, wie beispielsweise Greifzangen, Schneidwerkzeugen und Nähwerkzeugen, die über einen bzw. mehrere Trokare in das Körperinnere eines Patienten eingeführt werden. In der Regel kommen zwei bis vier, in den meisten Fällen drei, chirurgische Instrumente zum Einsatz. Neben diesen chirurgischen Instrumenten ist es erforderlich, dass eine Visualisierungseinheit vorhanden ist, die dem Chirurgen eine Beobachtung des Operationsgebiets ermöglicht. Eine solche Visualisierungseinheit ist regelmäßig eine Kamera bzw. ein Endoskop, die/das ebenfalls über einen Trokar in das Körperinnere des Patienten eingeführt wird. Üblicherweise erfolgt die Visualisierung über ein Endoskop, welches Aufnahmen des Operationsgebiets in 2D oder 3D auf einem externen Monitor darstellt. Im Stand der Technik sind zahlreiche Endoskope bekannt, bei welchen eine Visualisierungseinheit, wie einer Kamera, in ihr distales Ende integriert sind. Allgemein können Endoskope eine Kamera jedoch sowohl an ihrem distalen als auch an ihrem proximalen Ende aufweisen. Die mit dem Endoskop erzielten Aufnahmen werden über ein Bildweiterleitungssystem und einer Bildverarbeitungseinheit auf einem oder mehreren externen Monitoren abgebildet. Zahlreiche Endoskope werden im Stand der Technik beschrieben.

So beschreibt beispielsweise die WO2009/057117A2 ein Endoskop mit zwei bildgebenden Vorrichtungen. Die bildgebenden Vorrichtungen werden über einen Trokar in das Körperinnere geführt und über Klappen, die an dem Trokar befestigt sind, seitlich ausgeklappt und zwar winkelförmig von der Längsachse des Trokars zur Seite. Beide bildgebenden Vorrichtungen können mit einem unterschiedlichen Winkel ausgeschwenkt werden, so dass zwei verschiedene Aufnahmen erzielt werden können.

Die Nachteile der im Stand der Technik beschriebenen Kamerasysteme bzw. Endoskope ist, dass, selbst wenn zwei Kameras für die Aufnahmen des Operationsgebiets vorgesehen sind, diese Kameras nicht in jeder Konstellation alle chirurgischen Instrumente gleichzeitig abbilden können, bedingt durch die variierenden Positionen der chirurgischen Instrumente und der Position des Endoskops nahe am Operationsgeschehen sowie des Objektfeldwinkels (FoV, "Field of View"), wobei lediglich der unmittelbare Bereich des operativen Geschehens abgebildet wird. Wird ein chirurgisches Instrument aus dem Operationssichtfeld entfernt, wird es von der Kamera bzw. den Kameras nicht mehr erfasst und steht nicht mehr unter der visuellen Kontrolle des Chirurgen oder dessen Assistenten.

Der Erfindung liegt demnach die Aufgabe zugrunde, ein verbessertes Visualisierungssystem für minimal-invasive chirurgische Eingriffe, wie laparoskopische Eingriffe, zur Verfügung zu stellen, welches es dem Operateur mit einem einzigen Trokar oder Zugang ins Körperinnere bzw. ohne einen zusätzlichen Trokar oder Zugang ins Körperinnere ermöglicht, die Instrumente in vereinfachter Art und Weise zu koordinieren.
Diese Aufgabe wird durch die vorliegende Erfindung gemäß Anspruch 1 durch ein Endoskop und gemäß Anspruch 6 durch ein chirurgisches Robotersystem mit einem entsprechenden Endoskop gelöst.

Die vorliegende Erfindung stellt ein Endoskop mit einem Mehrkamerasystem für den Einsatz bei minimal-invasiven chirurgischen Eingriffen, wie der Laparoskopie, dar.

Ein erster Gegenstand der vorliegenden Erfindung betrifft ein Endoskop für die minimalinvasive Chirurgie, insbesondere zur Verwendung innerhalb eines chirurgischen Robotersystems, welches
eine Hauptträgereinrichtung, die sich im Wesentlichen über die gesamte Endoskoplänge von außen in das Körperinnere erstreckt, und welche am distalen Ende zumindest eine Beleuchtungseinheit und zwei Bildaufnahmeeinrichtungen aufweist, wobei die Bildaufnahmeeinrichtungen jeweils im Wesentlichen in der gleichen Ebene von der Hauptträgereinrichtung nach außen schwenkbar angeordnet sind,
einen Trokar, welcher den Zugang des Endoskops in das Körperinnere bewerkstelligt, und eine Zusatzträgereinrichtung aufweist, die an dem Trokar und/oder der Hauptträgereinrichtung vorgesehen ist, wobei die Zusatzträgereinrichtung an ihrem distalen Ende eine Zusatzbildaufnahmeeinrichtung aufweist, die von der Zusatzträgereinrichtung nach außen schwenkbar angeordnet ist und wobei die Zusatzbildaufnahmeeinrichtung eine Zusatzbeleuchtungseinheit und zumindest einen Zusatzbildsensor aufweist, welcher einen Überwachungsbereich aufweist, der die beiden Überwachungsbereiche der Bildaufnahmeeinrichtungen der Hauptträgereinrichtung umfasst,
dadurch gekennzeichnet, dass die Zusatzträgereinrichtung zwischen dem Trokar und der Hauptträgereinrichtung, insbesondere unmittelbar an der Hauptträgereinrichtung anliegt, wobei insbesondere sowohl die Hauptträgereinrichtung als auch die Zusatzträgereinrichtung zylinderförmig ausgebildet sind.

Die vorliegende Erfindung hat den Vorteil, dass durch die Bereitstellung und gleichzeitiger Nutzung von 2 bildgebenden Systemen, einer zumindest 2D-Übersichtskamera und einer 3D-Detailkamera, die über einen einzigen Trokar (auch Kombinations-Trokar) in das Körperinnere eines Patienten eingeführt werden, möglich ist, sowohl ein zumindest 2D-Übersichtsbild mit einem hohen Objektfeldwinkel (Weitwinkel von typischerweise > 90°) als auch ein 3D-Detailbild mit einem üblichen Objektfeldwinkel von bis zu 70° zu generieren. Dies ermöglicht es, während der gesamten Dauer eines minimal-invasiven chirurgischen, wie laparoskopischen, Eingriffs, das direkte Operationsgebiet sowie dessen weiteren Umkreis abzubilden. Auf diese Weise können alle chirurgischen Instrumente gleichzeitig abgebildet werden, auch wenn Sie sich, bedingt durch ihre variierenden Positionen und der Position der Kameras bzw. des Endoskops sowie des Objektfeldwinkels (FoV, "Field of View") außerhalb des Operationssichtfeldes der beiden Bildaufnahmeeinrichtungen am distalen Ende des Endoskop befinden, da die Zusatzbildaufnahmeeinrichtung auch Instrumente erfassen kann, welche sich außerhalb des Operation Sichtfeldes der beiden Bildaufnahmeeinrichtungen befinden. Dies kann beispielsweise der Fall sein, wenn ein chirurgisches Instrument zeitweise nicht benötigt "geparkt" wird. Dieses "Parken" erfolgt in den meisten Fällen außerhalb des direkten Operationsgeschehens und außerhalb des Operationssichtfeld, damit es bei dem Eingriff nicht im Wege ist. Erfindungsgemäß werden solche "geparkten" chirurgischen Elemente von der erfindungsgemäßen 2D-Übersichtskamera erfasst und stehen so kontinuierlich unter der visuellen Kontrolle des Chirurgen oder dessen Assistenten. Dadurch dass die als 2D-Übersichtskamera ausgebildete Zusatzbildaufnahmeeinrichtung und die als 3D-Detailkamera ausgebildete Bildaufnahmeeinrichtungen in Form von beispielsweise 2 Bildsensoren jeweils an einem Endoskop angeordnet sind, ist es für den Chirurgen unproblematisch, die Bildaufnahmen der 2D-Übersichtskamera und der 3D-Detailkamera über einen gemeinsamen oder getrennte Bildschirme zu verfolgen, wobei eine entsprechende Koordination für den Chirurgen dadurch einfach ist, da der

Überwachungsbereich der 2D-Übersichtskamera, welche gegebenenfalls eine 3D-Optik aufweisen kann, den Überwachungsbereich der 3D-Detailkamera umfasst bzw. größer ist als der Objektfeldwinkel der 3D-Detailkamera. Hierzu ist zu erwähnen, dass eine Anordnung von 2 separaten, völlig unabhängig orienterten Kameras mit einem überlappenden Überwachungsbereich, welche nicht an dem Endoskop angebracht sind, eine ungünstige Koordination für den Chirurgen erzeugen, so dass dieser gegebenenfalls aufgrund von 2 völlig unabhängigen Kameras "seekrank" werden kann. Diese Problematik wird durch das erfindungsgemäße Endoskop mit der 2D-Übersichtskamera und der zu dieser abgestimmten 3D-Detailkamera in einfacher Art und Weise gelöst.

Ferner wird durch die Beleuchtungseinheit an der Hauptträgereinrichtungen in Verbindung mit der Zusatzbeleuchtungseinheit an der Zusatzträgereinrichtung insbesondere für die 3D-Aufnahmen eine verbesserte Ausleuchtung erzielt, so dass die Bilder der 3D-Detailkamera qualitativ verbessert dargestellt werden können.

Gemäß einer bevorzugten Ausführungsform der Erfindung weist der Zusatzbildsensor eine Weitwinkeloptik auf, welcher im ausgeschwenkten Zustand nahe an dem distalen Ende des Trokars angeordnet ist.

Insbesondere ist es von Vorteil, wenn die beiden Bildaufnahmeeinrichtungen jeweils am distalen Ende der Hauptträgereinrichtung um eine Drehachse schwenkbar angeordnet sind, wobei die Schwenkachsen parallel zueinander in einer Ebene liegen, wodurch der konstruktive Aufwand minimiert ist.

Ferner ist es von Vorteil, wenn die Bildaufnahmeeinrichtungen mittels Gelenken jeweils sowohl um die Schwenkachse als auch um eine weitere Drehachse orthogonal zur Längserstreckung der Trägereinrichtung kippbar angeordnet sind, wobei die Drehbewegungen um die Schwenkachsen und die Drehachsen voneinander unabhängig entkoppelt sind.

Gemäß der vorliegenden Erfindung ist es somit möglich, bei Nutzung nur eines Trokars, die 3D-Detailkamera in 4 Freiheitsgraden unabhängig von der 2D-Übersichtskamera bewegt werden kann. 2D-Übersichtskamera und 3D-Detailkamera sind wiederum in 2 Freiheitsgraden miteinander gekoppelt, welche die Bewegungen in x- und y-Richtung (Pivotalbewegung des Trokars) um den Eintrittspunkt des Trokars in den Körper herum darstellen.

Ein zweiter Gegenstand der vorliegenden Erfindung betrifft ein chirurgisches Robotersystem mit zumindest einem Roboterarm, an welchem zumindest ein chirurgisches Instrument und/oder ein Endoskop für die minimalinvasive Chirurgie anordenbar ist, welches eine Hauptträgereinrichtung, die sich im Wesentlichen über die gesamte Endoskoplänge von außen in das Körperinnere erstreckt, und welche am distalen Ende zumindest eine Beleuchtungseinheit und zwei Bildaufnahmeeinrichtungen aufweist, wobei die Bildaufnahmeeinrichtungen jeweils im Wesentlichen in der gleichen Ebene von der Hauptträgereinrichtung nach außen schwenkbar angeordnet sind,
einen Trokar, welcher den Zugang des Endoskops in das Körperinnere bewerkstelligt, und eine Zusatzträgereinrichtung aufweist, die an dem Trokar und/oder der Hauptträgereinrichtung vorgesehen ist, wobei die Zusatzträgereinrichtung an ihrem distalen Ende eine Zusatzbildaufnahmeeinrichtung aufweist, die von der Zusatzträgereinrichtung nach außen schwenkbar angeordnet ist und wobei die Zusatzbildaufnahmeeinrichtung eine Zusatzbeleuchtungseinheit und zumindest einen Zusatzbildsensor aufweist, welcher einen Überwachungsbereich aufweist, der die beiden Überwachungsbereiche der Bildaufnahmeeinrichtungen der Hauptträgereinrichtung umfasst,
wobei eine Bildverarbeitungseinheit, welche sowohl mit den beiden Bildaufnahmeeinrichtungen als auch der Zusatzbildaufnahmeeinrichtung gekoppelt ist, und eine Visualisierungseinheit vorgesehen ist, welche 2D-Bilddaten und/oder 3D-Bilddaten der Bildaufnahmeeinrichtungen und/oder der Zusatzbildaufnahmeeinrichtung darstellt,
dadurch gekennzeichnet, dass die Zusatzträgereinrichtung zwischen dem Trokar und der Hauptträgereinrichtung, insbesondere unmittelbar an der Hauptträgereinrichtung anliegt, wobei insbesondere sowohl die Hauptträgereinrichtung als auch die Zusatzträgereinrichtung zylinderförmig ausgebildet sind.

Das erfindungsgemäße chirurgische Robotersystem weist insbesondere den Vorteil auf, dass die Bilddaten für den Chirurgen je nach Bedarf als 2D-Bilddaten und/oder 3D-Bilddaten dargestellt werden können, d.h., dass die Bilddaten der Übersichtskamera auch mit den Bilddaten der 3D-Detailkamera mithilfe der Bildbearbeitungseinheit gekoppelt werden können, um so dem Chirurgen eine stark verbesserte Übersicht durch eine einzige Bildabfolge auf der Visualisierungseinheit zu ermöglichen.

So ist es insbesondere von Vorteil, wenn der Zusatzbildsensor eine Weitwinkeloptik aufweist, welcher im ausgeschwenkten Zustand nahe an dem distalen Ende des Trokars angeordnet ist.

Weitere vorteilhafte Ausgestaltungen der Erfindung ergeben sich aus den Unteransprüchen.

Die gesamte Offenbarung der vorliegenden Erfindung bezieht sich demnach gleichermaßen sowohl auf die beiden Kamerasysteme als auch auf ein Endoskop umfassend diese beiden Kamerasysteme.

Bei minimal-invasiven chirurgischen Eingriffen, wie laparoskopischen Eingriffen, wird über einen Trokar ein Zugang in das Körperinnere eines Patienten (üblicherweise durch die Bauchdecke oder in den Brustraum) geschaffen. Durch einen solchen Trokar kann ein chirurgisches Instrument oder eine Kamera bzw. ein Endoskop in das Körperinnere geführt werden. Wie erwähnt, werden erfindungsgemäß über einen Trokar zwei Kameras gleichzeitig eingeführt. Da in der Regel für einen Eingriff 2 bis 4 chirurgische Instrumente und mindestens eine Kamera benötigt werden, sind 3 bis 5 Trokare erforderlich.

Rein beispielhaft wird die vorliegende Erfindung anhand der beigefügten Figuren erläutert. Es zeigt:
Figur 1 eine schematische Ansicht eines bestimmungsgemäßen Endoskops in einer bevorzugten Ausführungsform einer 3D-Detailkamera, welche an einem erfindungsgemäßen Endoskop angeordnet ist, welches mit einer Bildverarbeitungseinheit und einer Visualisierungseinheit eines chirurgischen Robotersystems verbunden ist, und
Figur 2 eine schematische Teilansicht einer weiteren Ausführungsform einer 3D-Detailkamera mit einer extern verbundenen Lichtquelle, welche an einem erfindungsgemäßen Endoskop angeordnet ist ,und
Figur 3 eine schematische Teilansicht einer weiteren, bevorzugten Ausführungsform einer 3D-Detailkamera, welche mittels einer reduzierten Anzahl der mechanischen Stellelemente realisierbar ist, welche an einem erfindungsgemäßen Endoskop angeordnet ist ,und
Figur 4 eine schematische Gesamtansicht der Verwendung der Visualisierungslösung in einem chirurgischen Robotersystem für den Einsatz in der minimal-invasiven Chirurgie, wie z.B. der Laparoskopie.

Die vorliegende Erfindung wird nachfolgend unter Bezugnahme auf die Figuren beschrieben:

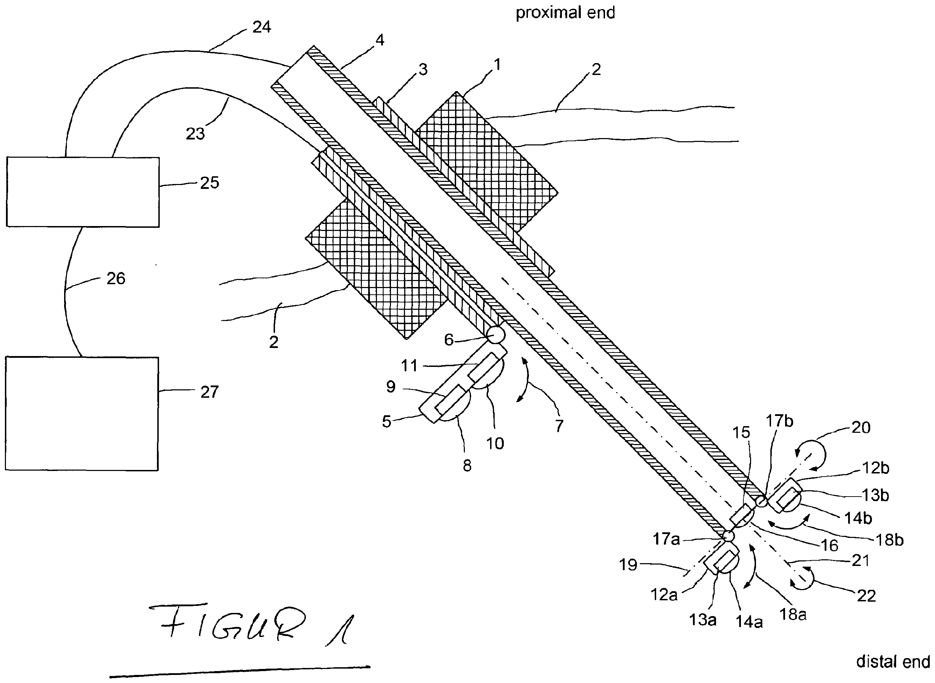

**Figur 1** zeigt ein Mehrkamerasystem in ein Endoskop integriert. Über einen Trokar 1 wird eine Durchführung durch das Körpergewebe 2 und somit ein Zugang in das Körperinnere eines Patienten geschaffen. Durch den Trokar 1 wird ein Zusatzträger 3 für eine 2D-Übersichtskamera in den Körper eingeführt. Der Zusatzträger 3 ist so gestaltet, dass dieser eine röhrenförmige Durchführung für einen rotationssymmetrischen, stabförmig gestalteten weiteren Hauptträger 4 für eine 3D-Detailkamera ermöglicht. Alternativ kann die röhrenförmige Durchführung auch für chirurgische Instrumente genutzt werden. An dem Zusatzträger 3 ist über ein Gelenk 6 ein Kamerahalter 5 so befestigt, dass dieser in wesentlichen um 90° zur Rotationsachse durch eine Schwenkbewegung 7 nach Durchführung durch den Trokar 1 ausgeklappt werden kann. Der Kamerahalter 5 trägt einen Zusatzbildsensor, bestehend aus Bildsensor 9 und Weitwinkeloptik-Abbildungsoptik 8. Um das Objektfeld auszuleuchten ist der Kamerahalter 5 weiterhin mit einer Zusatzbeleuchtungseinheit, bestehend aus einer Lichtquelle 11 und einer entsprechenden Weitwinkel-Abbildungsoptik 10, ausgestattet. Diese Weitwinkel-Abbildungsoptik 10 ist derart gestaltet, das das komplette vom Bildsensor 9 und der damit verbundenen Weitwinkel-Abbildungsoptik 8 erfasste Objektfeld, ausgeleuchtet wird. Der Kamerahalter 5 mit dem Zusatzbildsensor und der Zusatzbeleuchtungseinheit bilden zusammen die 2D-Übersichtskamera zur Erzeugung eines 2D-Übersichtsbildes. Vorzugsweise ist der Bildsensor 9 ausgeprägt als CCD- oder CMOS-Sensor mit einer Auflösung von 1920x1080 Bildpunkten oder höher.

Die aufgenommen Bilddaten werden über die Datenstrecke 23 einer Verarbeitungseinheit 25 zugeführt, welche die Bilddaten zur Darstellung aufbereitet und über eine weitere Datenstrecke 26 einer Visualisierungseinheit 27 zuführt. Die Visualisierungseinheit 27 kann sowohl 2D- als auch 3D-Bilddaten darstellen, beispielsweise getrennt, jedoch auch kombiniert in einem einzigen Bild bzw. einer einzigen Bildabfolge. Die Steuerung welche Bilddaten wie dargestellt werden sollen, erfolgt durch die Steuereinheit 32 je nach Wunsch des Operators bzw. Chirurgen.

Am Ende des rotationssymmetrischen Hauptträgers 4 befinden sich 2 Kameramodule bzw. zwei Bildaufnahmeeinrichtungen 12a, 13a, 14a, 12b, 13b, 14b bestehend insbesondere aus jeweils 2 Abbildungsoptiken 14a und 14b montiert auf 2 Kamerahaltern 12a und 12b. Die Kamerahalter 12a und 12b sind über die Gelenke 17a und 17b, welche die Schwenkachsen bilden, derart mit dem Hauptträger 4 verbunden, dass diese um 90° zur Rotationsachse des Hauptträgers 4 nach Einführung in den Körper in Schwenkrichtung 18a bzw. 18b ausgeklappt werden können. Um das Objektfeld zu beleuchten, ist an dem Ende des Hauptträgers 4 an dem auch die ausklappbaren Kamerahalter 12a und 12b befestigt sind, eine Beleuchtungseinheit, bestehend aus der Lichtquelle 15 und einer Abbildungsoptik 16, montiert. Die Kamerahalter 12a und 12b tragen weiterhin Bildaufnehmer, bestehend aus Bildsensoren 13a und 13b und Abbildungsoptiken 14a und 14b. Zusammen bilden diese beiden Bildaufnahmeeinrichtungen 12a, 13a, 14a, 12b, 13b, 14b die 3D-Detailkamera.
Die Beleuchtungseinheit, bestehend aus Lichtquelle 15 und Abbildungsoptik 16, kann vorzugsweise ausgeführt werden als direkte LED-Lichtquelle, derart gestaltet, das der Abstrahlwinkel der LED, in Verbindung mit einer geeigneten Abbildungsoptik 16, so gewählt wird, das das von beiden Bildsensoren 13a und 13b und den damit verbundenen Abbildungsoptiken 14a und 14b abgebildete Objektfeld, vollständig ausgeleuchtet wird. In einer abgewandelten Ausführungsform kann die Beleuchtungseinheit am proximalen Ende auch nur aus einer Abbildungsoptik 16 bestehen. Die Lichtquelle 30 ist dann außerhalb des Hauptträgers 4 und damit außerhalb des Patienten angeordnet. Die Steuerbefehle an die Lichtquelle 30 werden über die Datenstrecke 31 von der Verarbeitungseinheit 25 gesendet. Das Licht selbst wird dann über einen Lichtleiter in einen geeigneten Lichtleitungsmechanismus 28 eingekoppelt. Dieser Lichtleitungsmechanismus 28 ist zum Beispiel vorzugsweise ausgeführt als Lichtleitfaserbündel und führt das eingekoppelte Licht zur Abbildungsoptik 16. In einer weiteren Ausführungsform kann der Lichtleitungsmechanismus 28 auch durch geeignete Staboptiken realisiert werden.

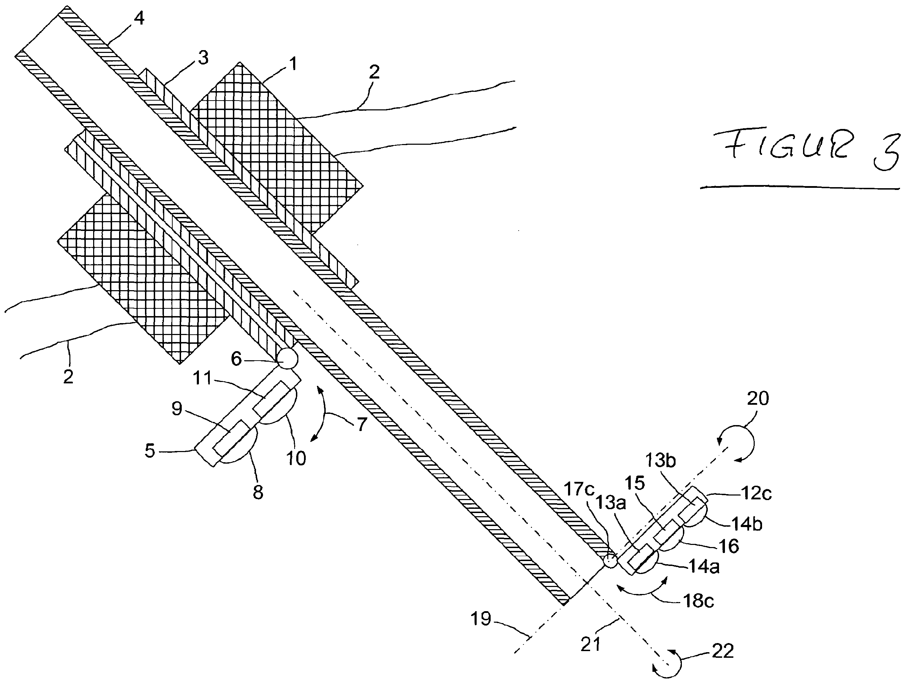

In Figur 3 befindet sich am Ende des rotationssymmetrischen Hauptträgers 4 ein Kamerahalter 12c, welcher beide Bildaufnahmeeinrichtungen 13a, 14a, 13b, 14b bestehend insbesondere aus jeweils 2 Abbildungsoptiken 14a und 14b montiert auf einem Kamerahalter 12c trägt. Die Lichtquelle 15 verbunden mit der Abbildungsoptik 16 ist in dieser Anordnung mittig zwischen den beiden Bildaufnahmeeinrichtungen 13a, 14a, 13b, 14b ebenfalls auf dem Kamerahalter 12c angeordnet. Der Kamerahalter 12c ist über das Gelenk 17c, welches die Schwenkachse bildet, derart mit dem Hauptträger 4 verbunden, dass dieser um 90° zur Rotationsachse des Hauptträgers 4 nach Einführung in den Körper in einer Schwenkbewegung 18c ausgeklappt werden kann.
Zusätzlich ist das Gelenk 17c so ausgeführt, das der Kamerahalter 12c um die orthogonal zur Rotationsachse 21 des Hauptträgers 4 liegende Drehachse 19 in die Richtung 20 um wenigstens +/- 90° geschwenkt werden kann.

Die aufgenommen Bilddaten werden über die Datenstrecke 24 einer Verarbeitungseinheit 25 zugeführt, welche die Bilddaten der 3D-Detailkamera zur Stereo-Darstellung aufbereitet und über die Datenstrecke 26 einer Visualisierungseinheit 27 zuführt. Die Visualisierungseinheit 27 kann sowohl 2D- als auch 3D-Bilddaten darstellen. Die Steuerung welche Bilddaten wie dargestellt werden sollen, erfolgt durch die Verarbeitungseinheit 25.

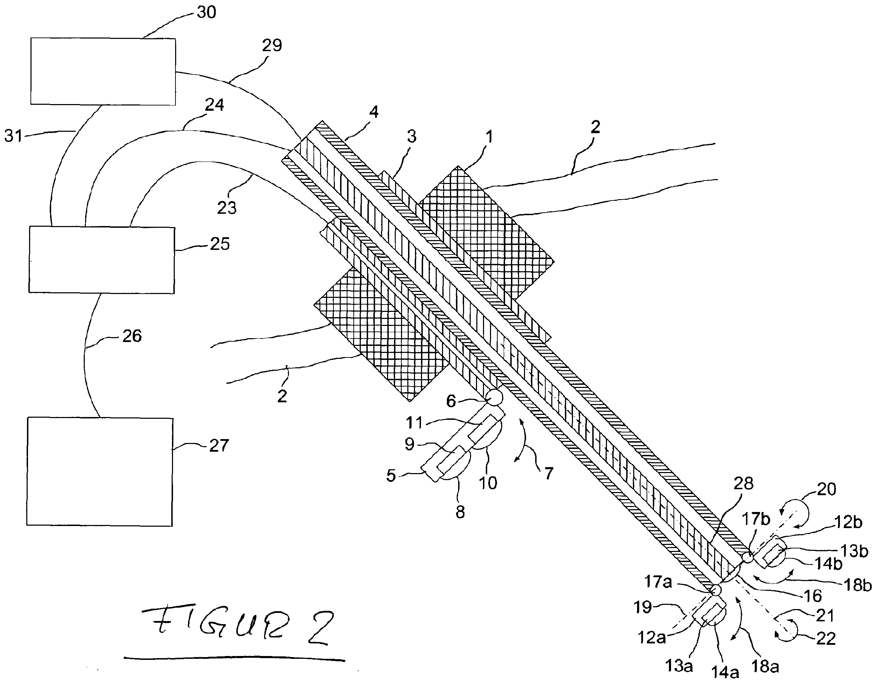

**Figur 2** verdeutlicht die Ausklappbewegungen der 3D-Detailkamera in Bezug auf Rotations- und Drehachse. Die Gelenke 17a und 17b (siehe Figur 1) ermöglichen eine Ausklappbewegung der Kamerahalter 12a und 12b (siehe Figur 1) um 90° ausgehend von der Rotationsachse 21 des Hauptträgers 4.

Die Gelenke 17a und 17b können weiterhin eine gleichgeschaltete Schwenkbewegung bzw. ein Kippen in etwa um +/- 90° der Kamerahalter 12a und 12b um eine Drehachse 19 orthogonal zur Rotationsachse des Hauptträgers 4 ermöglichen. Damit können ohne Veränderung der Position des Hauptträgers 4 entsprechende 3D-Bilder in einem Winkel zur Rotationsachse 21 des Hauptträgers 4 aufgenommen werden.

Diese gleichgeschaltete Schwenkbewegung der Kamerahalter 12a und 12b um die Drehachse 19 orthogonal zur Rotationsachse des Hauptträgers 4 ist vorteilhaft gegenüber Konstruktionen von Endoskopen aus dem Stand der Technik, bei denen die Rotationsachse und die optische Achse identisch sind, d.h. ein Blick "schräg nach oben" oder "schräg nach unten" erfordert ein Kippen des Endoskops um den entsprechenden Winkel und damit auch den erforderlichen Bewegungsraum. Hierdurch kann das Gewebe des Patienten strapaziert und auch verletzt werden. Ein solches herkömmliches Kippen des Endoskops ist bei dem erfindungsgemäßen Endoskop aufgrund des Schenkens der Kamerahalter um die Drehachse 19 nicht erforderlich. Alternativ kommen bei Endoskopen gemäß dem Stand der Technik verschiedene Optiken zum Einsatz, welche einen festen, von 0° verschiedenen Winkel, typischerweise 30°, aufweisen. Zum Wechsel der Endoskop-Optiken muss der Chirurg die OP unterbrechen, die Endoskop-Optik entfernen, eine andere Optik mit dem Endoskop verbinden und das Endoskop erneut in den Patienten durch den Endoskop-Trokar einführen.

Die Gelenke 17a und 17b ermöglichen weiterhin eine voneinander entkoppelte unabhängige Schwenkbewegung jeweils um im wesentlichen +/- 90° der Kamerahalter 12a und 12b um eine Drehachse 19 orthogonal zur Rotationsachse 21 des Hauptträgers 4. Damit können ohne Veränderung der Position des Hauptträgers 4 entsprechende 2D Bilder über einen größeren Objektfeldwinkel aufgenommen werden. Dazu werden die zwei 2D-Bilder nach Übertragung über die Datenstrecke 24 (siehe Figur 1) in einer Verarbeitungseinheit 25 (siehe Figur 1) so zusammengesetzt, dass als Ergebnis ein 2D-Bild über einen größeren Objektfeldwinkel auf der Visualisierungseinheit 27 (siehe Figur 1) dargestellt werden kann, was bei herkömmlichen Endoskopen nicht möglich ist. Falls die Visualisierungeinheit 27 auch für die Darstellung von 3-D-Bildern geeignet ist, so kann die Verarbeitungseinheit 25 auch insgesamt ein 3D-Bild errechnen, welches für den Chirurgen als dreidimensionales Bild auf der Visualisierungseinheit 27 dargestellt wird. Ein derartiges dreidimensionales Bild kann der Chirurg beispielsweise mittels eines optischen Hilfsmittels, wie z.B. einer Shutterbrille oder einer Polarisationsbrille auf der Visualisierungeinheit 27 erfassen. Alternativ ist die Visualisierungseinheit 27 mittels eines optischen Abbildungssystems derart gestaltet, das diese jeweils ein linkes Bild für das linke Auge und ein rechtes Bild für das rechte Auge projeziert. Auf die Verwendung zusätzlicher optischer Hilfsmittel, wie z.B. einer Shutterbrille oder einer Polarisationsbrille, kann bei dieser Ausführungsform verzichtet werden.

In Figur 3 trägt der Kamerahalter 12c beide Bildaufnahmeeinrichtungen 13a, 14a, 13b, 14b sowie die Beleuchtungseinrichtung, bestehend aus Lichtquelle 15 und Abbildungsoptik 16. Eine Schwenkbewegung mittels Gelenk 17c um im wesentlichen +/- 90° des Kamerahalters 12c um eine Drehachse 19 orthogonal zur Rotationsachse 21 des Hauptträgers 4 wirkt sich daher immer auf beide Bildaufnahmeeinrichtungen 13a, 14a, 13b, 14b sowie die Beleuchtungseinrichtung, bestehend aus Lichtquelle 15 und Abbildungsoptik 16, aus. Vorteilhaft in dieser Ausführungsform ist die optimale Anpassung der Abbildungsoptik 16 auf den durch die Bildaufnahmeeinrichtungen 13a, 14a, 13b, 14b aufgenommen Objektfeldwinkel, da in dieser Ausführungsform die Beleuchtungseinrichtung mit den Bildaufnahmeeinrichtungen mitschwenkt. In dieser Ausführungsform ist die Lichtquelle 15 vorzugsweise als LED-Beleuchtung ausgeführt.

Aus den Daten der 2D-Übersichtskamera kann in der Verarbeitungseinheit 25 die Position und Trajektorien der eingeführten Instrumente berechnet werden. Diese Trajektorieninformation kann als Zusatzinformation bei der Darstellung des 3D-Bildes auf der Visualisierungseinheit 27 in geeigneter Form, z.B. als Overlay-Darstellung eingeblendet werden.

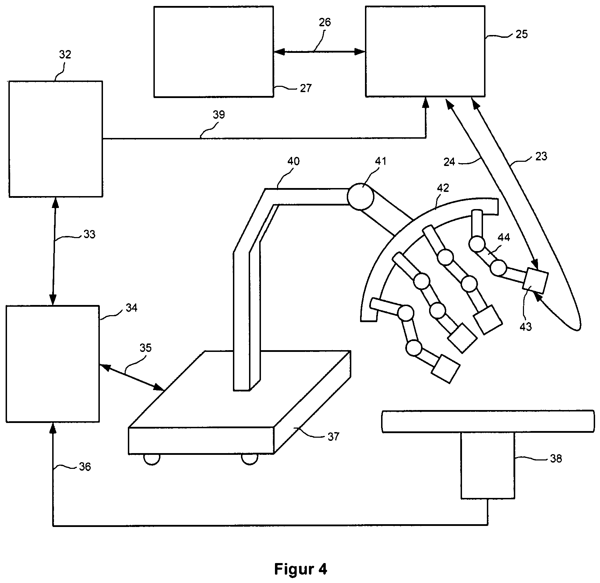

**Figur 4** zeigt die beispielhafte Verwendung des Mehrkamerasystems 43 in einem Telemanipulator bzw. Robotersystem. Die Aktorik (Stellantriebe) wird durch den Chirurgen über eine Anzeige- und Bedieneinheit 32 gesteuert. Die mittels der Anzeige- und Bedieneinheit 32 erzeugten Steuerbefehle werden über eine Datenübertragung 33 zu einer Steuereinheit 34 übermittelt. Diese ist über eine weitere Datenleitung 35 mit dem Robotereinheit 37 verbunden und, ausgestattet mit einem Tragarm 40, kann eine Bogenführung 42 entsprechend der Patientenposition auf dem OP-Tisch 38 über eine Gelenkmechanik 41 so vorpositioniert werden, dass der Roboterarm 44 ein optimale Positionierung des Mehrkamerasystem 43 ermöglicht. Die von der 2D-Übersichtskamera aufgenommen Bilddaten werden über die Datenstrecke 23 einer Verarbeitungseinheit 25 zugeführt, welche die Bilddaten zur Darstellung aufbereitet und über eine weitere Datenstrecke 26 einer Visualisierungseinheit 27 zuführt. Die Visualisierungseinheit 27 kann sowohl 2D- als auch 3D-Bilddaten darstellen, beispielsweise getrennt, jedoch auch kombiniert in einem einzigen Bild bzw. einer einzigen Bildabfolge. Die Steuerung, welche Bilddaten wie dargestellt werden sollen, erfolgt durch die Steuereinheit 32 je nach Wunsch des Operators bzw. Chirurgen. Die dazu von der Steuereinheit 32 erzeugten Steuerbefehle werden mittels der Datenstrecke 39 zur Verarbeitungseinheit 25 übertragen.
Gemäß Figur 1 werden durch zwei Bildaufnahmeeinrichtungen 12a, 13a, 14a, 12b, 13b, 14b bestehend insbesondere aus jeweils 2 Abbildungsoptiken 14a und 14b montiert auf 2 Kamerahaltern 12a und 12b zwei Bilder aus unterschiedlichen Positionen von einer Szene zum aufgenommen. Die aufgenommen Bilddaten werden über die Datenstrecke 24 einer Verarbeitungseinheit 25 zugeführt, welche die Bilddaten der 3D-Detailkamera zur Stereo-Darstellung aufbereitet und über die Datenstrecke 26 einer Visualisierungseinheit 27 zuführt. Die Visualisierungseinheit 27 kann sowohl 2D- als auch 3D-Bilddaten darstellen. Die Steuerung welche Bilddaten wie dargestellt werden sollen, erfolgt durch die Steuereinheit 32 je nach Wunsch des Operators bzw. Chirurgen. Die dazu von der Steuereinheit 32 erzeugten Steuerbefehle werden mittels der Datenstrecke 39 zur Verarbeitungseinheit 25 übertragen.

Minimal-invasive chirurgische Eingriffe, wie laparoskopische Operationen, werden häufig über chirurgische Manipulatoren, auch Telemanipulatoren oder Robotersysteme, durchgeführt. Das erfindungsgemäße Endolskop und chirurgische Instrument sind in solchen Telemanipulatoren bzw. Robotersystemen einsetzbar.

Die vorliegende Erfindung ist dennoch nicht auf den Einsatz des erfindungsgemäßen Endoskops bzw. des erfindungsgemäßen chirurgischen Instruments in einem Telemanipulator oder Robotersystem für den Einsatz in der minimal-invasiven Chirurgie beschränkt, sondern ist auch unabhängig von einem solchen System im medizinischen Anwendungsbereich einsetzbar.

## Patentansprüche

1. Endoskop für die minimalinvasive Chirurgie, insbesondere zur Verwendung innerhalb eines chirurgischen Robotersystems, welches
eine Hauptträgereinrichtung (4), die sich im Wesentlichen über die gesamte Endoskoplänge von außen in das Körperinnere erstreckt, und welche am distalen Ende zumindest eine Beleuchtungseinheit (15, 16) und zwei Bildaufnahmeeinrichtungen (12a, 13a, 14a, 12b, 13b, 14b; 12c) aufweist, wobei die Bildaufnahmeeinrichtungen (12a, 13a, 14a, 12b, 13b, 14b; 12c) jeweils im Wesentlichen in der gleichen Ebene von der Hauptträgereinrichtung (4) nach außen schwenkbar angeordnet sind,
einen Trokar (1), welcher den Zugang des Endoskops in das Körperinnere bewerkstelligt, und
eine Zusatzträgereinrichtung (3) aufweist, die an dem Trokar (1) und/oder der Hauptträgereinrichtung (4) vorgesehen ist, wobei die Zusatzträgereinrichtung (3) an ihrem distalen Ende eine Zusatzbildaufnahmeeinrichtung (8, 9, 10, 11) aufweist, die von der Zusatzträgereinrichtung (3) nach außen schwenkbar angeordnet ist und wobei die Zusatzbildaufnahmeeinrichtung (7, 8, 9, 10) eine Zusatzbeleuchtungseinheit (10, 11) und zumindest einen Zusatzbildsensor (8, 9) aufweist, welcher einen Überwachungsbereich aufweist, der die beiden Überwachungsbereiche der Bildaufnahmeeinrichtungen (12a, 13a, 14a, 12b, 13b, 14b; 12c) der Hauptträgereinrichtung (4) umfasst **dadurch gekennzeichnet, dass**
die Zusatzträgereinrichtung (3) zwischen dem Trokar (1) und der Hauptträgereinrichtung (4), insbesondere unmittelbar an der Hauptträgereinrichtung (4) anliegt, wobei insbesondere sowohl die Hauptträgereinrichtung (4) als auch die Zusatzträgereinrichtung (3) zylinderförmig ausgebildet sind.

2. Endoskop nach Anspruch 1, **dadurch gekennzeichnet, dass** der Zusatzbildsensor (8, 9) eine Weitwinkeloptik (8) aufweist, welcher im ausgeschwenkten Zustand nahe an dem distalen Ende des Trokars (1) angeordnet ist.

3. Endoskop nach Anspruch 1 oder 2, **dadurch gekennzeichnet, dass** die beiden Bildaufnahmeeinrichtungen (12a, 13a, 14a, 12b, 13b, 14b; 12c) jeweils am distalen Ende der Hauptträgereinrichtung (4) um eine Schwenkachse (17a, 17b; 17c) schwenkbar angeordnet sind, wobei die Schwenkachsen (17a, 17b; 17c) parallel zueinander in einer Ebene liegen.

4. Endoskop nach einem der Ansprüche 1 bis 3, **dadurch gekennzeichnet, dass** die Bildaufnahmeeinrichtungen (12a, 13a, 14a, 12b, 13b, 14b; 12c) mittels Gelenken (17a, 17b; 17c) jeweils sowohl um die Schwenkachse (17a, 17b; 17c) als auch um eine weitere Drehachse (19) orthogonal zur Längserstreckung der Trägereinrichtung (4) kippbar angeordnet sind, wobei die Drehbewegungen um die Schwenkachsen (17a, 17b; 17c) und die Drehachsen (19) voneinander unabhängig entkoppelt sind.

5. Chirurgisches Robotersystem mit zumindest einem Roboterarm, an welchem zumindest ein chirurgisches Instrument und/oder ein Endoskop für die minimalinvasive Chirurgie anordenbar ist, welches
eine Hauptträgereinrichtung (4), die sich im Wesentlichen über die gesamte Endoskoplänge von außen in das Körperinnere erstreckt, und welche am distalen Ende zumindest eine Beleuchtungseinheit (15, 16) und zwei Bildaufnahmeeinrichtungen (12a, 13a, 14a, 12b, 13b, 14b; 12c) aufweist, wobei die Bildaufnahmeeinrichtungen (12a, 13a, 14a, 12b, 13b, 14b; 12c) jeweils im Wesentlichen in der gleichen Ebene von der Hauptträgereinrichtung (4) nach außen schwenkbar angeordnet sind,
einen Trokar (1), welcher den Zugang des Endoskops in das Körperinnere bewerkstelligt, und
eine Zusatzträgereinrichtung (3) aufweist, die an dem Trokar (1) und/oder der Hauptträgereinrichtung (4) vorgesehen ist, wobei die Zusatzträgereinrichtung (3) an ihrem distalen Ende eine Zusatzbildaufnahmeeinrichtung (8, 9, 10, 11) aufweist, die von der Zusatzträgereinrichtung (3) nach außen schwenkbar angeordnet ist und wobei die Zusatzbildaufnahmeeinrichtung (8, 9, 10, 11) eine Zusatzbeleuchtungseinheit (10, 11) und zumindest einen Zusatzbildsensor (8, 9) aufweist, welcher einen Überwachungsbereich aufweist, der die beiden Überwachungsbereiche der Bildaufnahmeeinrichtungen (12a, 13a, 14a, 12b, 13b, 14b; 12c) der Hauptträgereinrichtung (4) umfasst,
wobei eine Bildverarbeitungseinheit (25), welche sowohl mit den beiden Bildaufnahmeeinrichtungen (12a, 13a, 14a, 12b, 13b, 14b; 12c) als auch der Zusatzbildaufnahmeeinrichtung (8, 9, 10, 11) gekoppelt ist, und eine Visualisierungseinheit (27) vorgesehen ist, welche 2D-Bilddaten und/oder 3D-Bilddaten der Bildaufnahmeeinrichtungen (12a, 13a, 14a, 12b, 13b, 14b; 12c) und/oder der Zusatzbildaufnahmeeinrichtung (8, 9, 10, 11) darstellt,
**dadurch gekennzeichnet, dass**
die Zusatzträgereinrichtung (3) zwischen dem Trokar (1) und der Hauptträgereinrichtung (4), insbesondere unmittelbar an der Hauptträgereinrichtung (4) anliegt, wobei insbesondere sowohl die Hauptträgereinrichtung (4) als auch die Zusatzträgereinrichtung (3) zylinderförmig ausgebildet sind.

6. Robotersystem nach Anspruch 5, **dadurch gekennzeichnet, dass** der Zusatzbildsensor (8, 9) eine Weitwinkeloptik (8) aufweist, welcher im ausgeschwenkten Zustand nahe an dem distalen Ende des Trokars (1) angeordnet ist.

7. Robotersystem nach Anspruch 5 oder 6, **dadurch gekennzeichnet, dass** die beiden Bildaufnahmeeinrichtungen (12a, 13a, 14a, 12b, 13b, 14b; 12c) jeweils am distalen Ende der Hauptträgereinrichtung (4) um eine Schwenkachse (17a, 17b; 17c) schwenkbar angeordnet sind, wobei die Schwenkachsen (17a, 17b) parallel zueinander in einer Ebene liegen.

8. Robotersystem nach einem der Ansprüche 5 bis 7, **dadurch gekennzeichnet, dass** die Bildaufnahmeeinrichtungen (12a, 13a, 14a, 12b, 13b, 14b; 12c) mittels Gelenken (17a, 17b; 17c) jeweils sowohl um die Schwenkachse (17a, 17b; 17c) als auch um eine weitere Drehachse (19) orthogonal zur Längserstreckung der Trägereinrichtung (4) kippbar angeordnet sind, wobei die Drehbewegungen um die Schwenkachsen (17a, 17b; 17c) und die Drehachsen (19) voneinander unabhängig entkoppelt sind.

## Claims

1. Endoscope for minimally invasive surgery, especially for use within a surgical robot system, which comprises
a main support device (4), which basically extends over the entire length of the endoscope from the outside to the interior of the body, and which comprises at its distal end at least one lighting unit (15, 16) and two imaging devices (12a, 13a, 14a, 12b, 13b, 14b; 12c), wherein each of the imaging devices (12a, 13a, 14a, 12b, 13b, 14b; 12c) is basically arranged in such a way that it can be rotated to the outside on the same level as the main support device (4),
a trocar (1), by means of which the endoscope can enter the body, and
an additional support device (3), which is provided at the trocar (1) and/or the main support device (4), wherein the additional support device (3) comprises at its distal end an additional imaging device (8, 9, 10, 11), which can be rotated from the additional support device (3) to the outside and wherein the additional imaging device (7, 8, 9, 10) comprises an additional lighting unit (10, 11) and at least an additional image sensor (8, 9), which comprises a monitoring area, which encompasses the two monitoring areas of the imaging devices (12a, 13a, 14a, 12b, 13b, 14b; 12c) of the main support device (4),
**characterized in that**
the additional support device (3) is located between the trocar (1) and the main support device (4), especially resting directly at the main support device (4), wherein in particular the main support device (4) and the additional support device (3) have a cylindrical design.

2. Endoscope according to Claim 1, **characterized in that** the additional image sensor (8, 9) comprises a wide-angle lens (8) which is located near the distal end of the trocar (1) when pivoted.

3. Endoscope according to Claim 1 or 2, **characterized in that** each of the two imaging devices (12a, 13a, 14a, 12b, 13b, 14b; 12c) can be rotated about a rotation axis (17a, 17b; 17c) at the distal end of the main support device (4), wherein the rotation axes (17a, 17b; 17c) are aligned in a plane parallel to one another.

4. Endoscope according to any one of claims 1 to 3, **characterized in that** each of the imaging devices (12a, 13a, 14a, 12b,13b; 12c) is arranged in such a way that it can be tilted by means of joints (17a, 17b; 17c) about the rotation axis (17a, 17b; 17c), as well as about a further pivot axis (19), in orthogonal direction toward the longitudinal extension of the support device (4), wherein the rotary movements about the rotation axes (17a, 17b; 17c) and the rotation axes (19) are decoupled independently of one another.

5. Surgical robot system having at least one robotic arm on which at least one surgical instrument and/or one endoscope for minimally invasive surgery can be arranged, which comprises
a main support device (4) that basically extends over the entire length of the endoscope from the outside to the interior of the body, and which comprises at its distal end at least one lighting unit (15, 16) and two imaging devices (12a, 13a, 14a, 12b, 13b, 14b; 12c), wherein each of the imaging devices (12a, 13a, 14a, 12b, 13b, 14b; 12c) is basically arranged in such a way that it can be rotated to the outside on the same level as the main support device (4),
a trocar (1), by means of which the endoscope can enter the body,
and an additional support device (3), which is provided at the trocar (1) and/or the main support device (4), wherein the additional support device (3) comprises at its distal end an additional imaging device (8, 9, 10, 11), which can be rotated from the additional support device (3) to the outside and wherein the additional imaging device (7, 8, 9, 10) comprises an additional lighting unit (10, 11) and at least an additional image sensor (8, 9), which comprises a monitoring area, which encompasses the two monitoring areas of the imaging devices (12a, 13a, 14a, 12b, 13b, 14b; 12c) of the main support device (4),
wherein provision has been made for an image processing unit (25), which is connected with the two imaging devices (12a, 13a, 14a, 12b, 13b, 14b; 12c) and the additional imaging device (8, 9, 10, 11), and a visualization unit (27), which represents 2D image data and/or 3D image data of the imaging devices (12a, 13a, 14a, 12b, 13b, 14b; 12c) and/or the additional imaging device (8, 9, 10, 11),
**characterized in that**
the additional support device (3) is located between the trocar (1) and the main support device (4), especially resting directly at the main support device (4), wherein in particular the main support device (4) and the additional support device (3) have a cylindrical design.

6. Robot system according to Claim 5, **characterized in that** the additional image sensor (8, 9) comprises a wide-angle lens (8) which is located near the distal end of the trocar (1) when pivoted.

7. Robot system according to Claim 5 or 6, **characterized in that** the two imaging devices (12a, 13a, 14a, 12b, 13b, 14b; 12c) are arranged at the distal end of the main support device (4), respectively, in such a manner that they can be rotated about a rotation axis (17a, 17b; 17c), wherein the rotation axes (17a, 17b) are aligned in a plane parallel to one another.

8. Robot system according to any one of claims 5 to 7, **characterized in that** each of the imaging devices (12a, 13a, 14a, 12b, 13b, 14b; 12c) is arranged in such a way that it can be tilted by means of joints (17a, 17b; 17c) about the rotation axis (17a, 17b; 17c), as well as about a further rotation axis (19), in orthogonal direction toward the longitudinal extension of the support device (4), wherein the rotary movements about the rotation axes (17a, 17b; 17c) and the rotation axes (19) are decoupled independently of one another.

## Revendications

1. Endoscope pour la chirurgie mini-invasive, en particulier destiné à être dans un système de robot chirurgical, lequel présente
un dispositif de support principal (4), qui s'étend sensiblement sur la totalité de la longueur de l'endoscope depuis l'extérieur jusqu'à l'intérieur du corps et qui comporte, au niveau de l'extrémité distale, au moins une unité d'éclairage (15, 16) et deux dispositifs d'enregistrement d'images (12a, 13a, 14a, 12b, 13b, 14b ; 12c), dans lequel les dispositifs d'enregistrement d'images (12a, 13a, 14a, 12b, 13b, 14b; 12c) sont disposés de manière à pouvoir pivoter respectivement sensiblement dans le même plan depuis le dispositif de support principal (4) vers l'extérieur,
un trocart (1), qui permet l'accès de l'endoscope à l'intérieur du corps, et
un dispositif de support supplémentaire (3), qui est prévu au niveau du trocart (1) et/ou du dispositif de support principal (4), dans lequel le dispositif de support supplémentaire (3) comporte, au niveau de son extrémité distale, un dispositif d'enregistrement d'images supplémentaire (8, 9, 10, 11), qui est disposé de manière à pouvoir pivoter depuis le dispositif de support supplémentaire (3) vers l'extérieur et dans lequel le dispositif d'enregistrement d'images supplémentaire (7, 8, 9, 10) comporte une unité d'éclairage supplémentaire (10, 11) et au moins un capteur d'image supplémentaire (8, 9), qui comprend une zone de surveillance contenant les deux zones de surveillance des dispositifs d'enregistrement d'images (12a, 13a, 14a, 12b, 13b, 14b; 12c) du dispositif de support principal (4),
**caractérisé en ce que**
le dispositif de support supplémentaire (3) repose entre le trocart (1) et le dispositif de support principal (4), en particulier directement au niveau du dispositif de support principal (4), dans lequel en particulier à la fois le dispositif de support principal (4) et le dispositif de support supplémentaire (3) sont réalisés de manière à présenter une forme cylindrique.

2. Endoscope selon la revendication 1, **caractérisé en ce que** le capteur d'image supplémentaire (8, 9) comporte une optique à angle large (8), qui est disposée, dans l'état sorti par pivotement, à proximité de l'extrémité distale du trocart (1).

3. Endoscope selon la revendication 1 ou 2, **caractérisé en ce que** les deux dispositifs d'enregistrement d'images (12a, 13a, 14a, 12b, 13b, 14b; 12c) sont disposés respectivement au niveau de l'extrémité distale du dispositif de support principal (4) de manière à pouvoir pivoter autour d'un axe de pivotement (17a, 17b ; 17c), les axes de pivotement (17a, 17b ; 17c) étant parallèles entre eux dans un plan.

4. Endoscope selon l'une quelconque des revendications 1 à 3, **caractérisé en ce que** les dispositifs d'enregistrement d'images (12a, 13a, 14a, 12b, 13b, 14b ; 12c) sont disposés de manière à pouvoir basculer au moyen d'articulations (17a, 17b ; 17c) respectivement à la fois autour de l'axe de pivotement (17a, 17b ; 17c) et autour d'un autre axe de rotation (19) de manière orthogonale par rapport à l'extension longitudinale du dispositif de support (4), dans lequel les déplacements par rotation autour des axes de pivotement (17a, 17b; 17c) et des axes de rotation (19) sont découplés indépendamment les uns des autres.

5. Système de robot chirurgical avec au moins un bras de robot équipé d'au moins un instrument chirurgical et/ou un endoscope pour la chirurgie mini-invasive, lequel présente
un dispositif de support principal (4), qui s'étend sensiblement sur toute la longueur de l'endoscope depuis l'extérieur jusqu'à l'intérieur du corps et qui comporte, au niveau de l'extrémité distale, au moins une unité d'éclairage (15, 16) et deux dispositifs d'enregistrement d'images (12a, 13a, 14a, 12b, 13b, 14b; 12c), dans lequel les dispositifs d'enregistrement d'images (12a, 13a, 14a, 12b, 13b, 14b; 12c) sont disposés respectivement sensiblement dans le même plan de manière à pouvoir pivoter depuis le dispositif de support principal (4) vers l'extérieur,
un trocart (1), qui permet l'accès de l'endoscope à l'intérieur du corps, et
un dispositif de support supplémentaire (3), qui est prévu au niveau du trocart (1) et/ou du dispositif de support principal (4), dans lequel le dispositif de support supplémentaire (3) comporte, au niveau de son extrémité distale, un dispositif d'enregistrement d'images supplémentaire (8, 9, 10, 11), qui est disposé de manière à pouvoir pivoter depuis le dispositif de support supplémentaire (3) vers l'extérieur et dans lequel le dispositif d'enregistrement d'images supplémentaire (8, 9, 10, 11) comporte une unité d'éclairage supplémentaire (10, 11) et au moins un capteur d'image supplémentaire (8, 9), lequel comprend une zone de surveillance contenant les deux zones de surveillance des dispositifs d'enregistrement d'images (12a, 13a, 14a, 12b, 13b, 14b; 12c) du dispositif de support principal (4),
dans lequel sont prévues une unité de traitement d'images (25), qui est couplée à la fois aux deux dispositifs d'enregistrement d'images (12a, 13a, 14a, 12b, 13b, 14b ; 12c) et au dispositif d'enregistrement d'images supplémentaire (8, 9, 10, 11), et une unité de visualisation (27), qui représente des données d'image 2D et/ou des données d'image 3D des dispositifs d'enregistrement d'images (12a, 13a, 14a, 12b, 13b, 14b ; 12c) et/ou du dispositif d'enregistrement d'images supplémentaire (8, 9, 10, 11), **caractérisé en ce que**
le dispositif de support supplémentaire (3) repose entre le trocart (1) et le dispositif de support principal (4), en particulier directement au niveau du dispositif de support principal (4), dans lequel en particulier à la fois le dispositif de support principal (4) et le dispositif de support supplémentaire (3) sont réalisés de manière à présenter une forme cylindrique.

6. Système de robot selon la revendication 5, **caractérisé en ce que** le capteur d'image supplémentaire (8, 9) comporte une optique à angle large (8), qui est disposée, dans l'état sorti par pivotement, à proximité de l'extrémité distale du trocart (1).

7. Système de robot selon la revendication 5 ou 6, **caractérisé en ce que** les deux dispositifs d'enregistrement d'images (12a, 13a, 14a, 12b, 13b, 14b; 12c) sont disposés respectivement au niveau de l'extrémité distale du dispositif de support principal (4) de manière pouvoir pivoter autour d'un axe de pivotement (17a, 17b ; 17c), les axes de pivotement (17a, 17b) étant parallèles entre eux dans un plan.

8. Système de robot selon l'une quelconque des revendications 5 à 7, **caractérisé en ce que** les dispositifs d'enregistrement d'images (12a, 13a, 14a, 12b, 13b, 14b; 12c) sont disposés de manière à pouvoir basculer au moyen d'articulations (17a, 17b ; 17c) respectivement à la fois autour de l'axe de pivotement (17a, 17b ; 17c) et autour d'un autre axe de rotation (19) de manière orthogonale par rapport à l'extension longitudinale du dispositif de support (4), dans lequel les déplacements par rotation autour des axes de pivotement (17a, 17b ; 17c) et des axes de rotation (19) sont découplés indépendamment les uns des autres.
